# EUROPEAN PATENT APPLICATION

(11) **EP 4 111 864 A1**
(43) Date of publication of application: **04.01.2023**
(21) Application number: 21461557.7
(22) Date of filing: 28.06.2021
(51) Int. Cl.: A01N 63/22, A01N 63/23, A01N 63/27, A01N 63/38, C05F 11/08, C05G 5/10, C05G 5/12, C12N 1/14, C12N 1/20

(54) **A BIOFORMULATION BASED ON GRANULATED OATS HULL AND A METHOD FOR ITS PREPARATION**

(71) Applicant: International Chemical Company S.A., 66-200 Swiebodzin (PL)
(72) Inventor: WYSOCZANSKA, Anna, 66-200 Swiebodzin (PL); WOLNA-MARUWKA, Agnieszka, 60-465 Poznan (PL); NIEWIADOMSKA, Alicja, 62-020 Swarzedz (PL); KAMINSKI, Adam, 66-218 Lubrza (PL)
(74) Representative: AOMB Polska Sp. z.o.o.

(57) **Abstract**

The invention relates to a bioformulation containing a carrier granulate based on granulated, purified oat hull with addition of molasses and at least one microbiological additive selected from fungi of *Trichoderma* genus and bacteria of the *Bacillus* and *Pseudomonas* genus. The invention further relates to a method for the preparation of said bioformulation, and a method for providing said bioformulation as fertilizer. The invention also relates to a carrier granulate based on granulated, purified oat hull with the addition of molasses and a method of its production.

## Description

The invention relates to a bioformulation comprising a carrier granulate based on granulated, purified oat hull with addition of molasses and at least one microbiological additive selected from fungi of genus *Trichoderma* and bacteria of the genus *Bacillus* and *Pseudomonas.* The invention further relates to a method for the preparation of said organic bioformulation, and a method for providing said bioformulation as a fertilizer. The invention also relates to a carrier granulate based on granulated, purified oat hull with the addition of molasses and a method of its production.

### Prior art

Currently, with the growing awareness of society, it is becoming more important to look for formulations used in agriculture, alternative to chemical means of protection, and mineral fertilization, the purpose of which is to increase the fertility of the soil and improve its sanitary condition and yield. One of the possibilities of increasing crop yields is the use of appropriate agrotechnical treatments, organic fertilization, and the use of biological plant protection based on the use of microorganisms. Preparations used in agriculture with addition of microorganisms are often characterized by a slower effect than their artificial analogs but they do not adversely affect the biological balance of the soil, and therefore they significantly fit into the current agricultural policy of the EU (Directive 2009/128 / EC) on integrated plant protection.

The oat hull contains significant amounts of dry matter (over 90%), carbohydrates, fiber, minerals, polyphenols and it has high antioxidant activity. It is also rich in essential unsaturated fatty acids, of which it contains more than other crops, and protein, which has a high biological value. Moreover, the content of minerals in oats (P, K, Mg, Ca, Fe, Zn, Mn, Si) is higher than in other grains. In oat crops, stem-base diseases and soil-borne diseases occur very rarely or not at all, because oats secrete specific organic substances that are fungicidal against soil pathogens. The phytosanitary properties of oats result from the presence of a saponin - avenacin. This compound is characterized by a strong fungotoxic effect limiting the development of various species of fungi. Due to its chemical composition, oats exhibit natural protection not only against pathogenic microorganisms but also against weeds.
Document AU2020220068 relates to a granular composition containing at least one microorganism, for example, a fungus selected from the group consisting of multiple species including fungi of genus *Trichoderma,* optionally in combination with a bacterium selected from the group comprising many different species including bacteria of the genus *Pseudomonas* or *Bacillus,* two or more plant powders (selected from the group comprising plant meals, and in some embodiments, oat bran) and a biodegradable water absorbing agent (selected from the group consisting of starches, cross-linked polymers, and peat). The composition is produced using a substrate that provides the appropriate ballistic properties and ensures effective spreading of the composition. Such a substrate may be, inter alia, oat grain. The composition may also include saccharide (e.g. molasses) and processed cereals e.g. rolled oats or puffed oats. The composition is used, among others, as fertilizer.
EP3544447 relates to a substrate (e.g. fertilizer granulate made from pressed oats containing an active ingredient selected from the group including many species of microorganisms, including but not limited to, bacteria e.g. bacteria of the genus *Pseudomonas* or *Bacillus* and fungi of the genus *Trichoderma,* and their combinations) and carrier particles (containing natural waxes having a melting point> 50 °C, synthetic or mineral, polymers, biopolymers (such as starches, chitin and natural waxes)) comprising an organic component on their at least one outer surface. According to the invention, the active ingredient is contained inside as well as on the surFace of the carrier particles, while the carrier particles are in dry form and carry at least a surFace electrostatic charge and are uniformly distributed on the surFace of the substrate.
Patent application EP3277826 relates to a method of producing a product by contacting at least one sugar with a fermentation composition comprising cellular material treated using a lysis process. According to the invention, at least one sugar is produced by saccharifying biomass comprising lignocellulosic material (including organic materials selected from the group including but not limited to, oat hulls and molasses). The cellular material treaded using the lysis process may comprise fungal cells of the genus *Trichoderma* (most preferably *Trichoderma reesei*). The fermentation composition may comprise a fermenting agent selected from the group including bacteria, e.g. bacteria of the genus *Pseudomonas* or *Bacillus.* This document also relates to a product produced by said method and comprising said ingredients. The product may be in the form of pellets but only if it is intended for use as a biofuel.
CA2973961 relates to a process for the preparation of an environmental modifying product including preparing an aqueous mixture containing at least one microorganism (e.g. at least one bacterium or *Trichoderma* fungus), at least a starch component (e.g. oats) and water; culturing of a microorganism; adding at least one sugar (e.g. molasses) to the culture; leaving the mixture under fermentation conditions; adding a carrier (e.g. zeolite); separating the carrier from the aqueous mixture and drying the carrier.
Document LU92687 relates to a fertilizer comprising a mineral fertilizer, plant matter (e.g. beet molasses) and at least one microorganism selected from *Bacillus amyloliquefaciens, B. megaterium, B. mucliagenosus, Trichoderma asperellum* and *T. atroviride.* The document also relates to a method of making such a fertilizer including mixing ingredients (except the microorganisms), injecting the microorganisms, pressing into sheets, milling the sheets, and granulating.
Patent RU2687023 describes a preparation containing a bacterial strain of *Pediococcus pentosaceus* in the form of bacterial cells combined with inert ingredients, optionally dried and/or ground. The inert component may be, among others, oat hulls, and the preparation may additionally contain molasses.
Document US10683238 relates to a granular composition containing a cellulosic nutrient (e.g., oat straw); an ingredient containing a mixture of microorganisms, preferably at least one species of bacteria (e.g. belonging to the genus *Bacillus* and *Pseudomonas*) and at least one species of fungi (e.g. belonging to the genus *Trichoderma*), as well as an activator triggering the growth of at least one microorganism in a mixture of microorganisms. The composition may also contain carbon compounds as an energy source, e.g. beet molasses.
US20200131096 discloses a bio-fertilizer composition containing a group of microorganisms (selected from the group including, but not limited to, bacteria belonging to the genus *Bacillus* and fungi belonging to the genus *Trichoderma*) in a culture medium comprising at least one carbon source (e.g. molasses); a first additive comprising a complexing agent in form of organic acid and a second additive of nitrogen source, potassium source, chelated metal, salt and water.
US20200337314 describes a microbiological composition containing a group of microorganisms (e.g. *Bacillus* bacteria and fungi) in a culture medium comprising at least one carbon source (e.g. molasses). The composition is used as a fertilizer for plants.
Patent application BR102017022427 relates to a bioactivated mixture containing molasses, oat bran, wheat and rice flour, bone meal, bird eggs, ground coal. The mixture may be used as a bio-fertilizer
CN105110826 relates to a method of producing a fertilizer containing microorganisms having an antibiotic activity, based on the fermentation of a base material using cellulololytic microorganisms (selected from the group including *Cellulomonas flavigena, Streptomycessp., Nocardia cellulans, Trichoderma viride* and *Clostridium thermocellum*) and at least one bacterium selected from the group including *Bacillus natto, Saccharomyces cereviseae* and *Lactobacillus plantarum,* and the base material is a waste organic material selected from the group including, among others, oat hulls.
DE1492926 discloses a method of producing fertilizer from discrete carbohydrate particles by treating a carbohydrate material (selected from the group including but not limited to oat hulls) at elevated pressure and temperature with gaseous nitrogen containing compound ((selected from the group including but not limited to ammonia, acyl derivatives of ammonia, primary, secondary and tertiary amines). Various additives, including molasses, may be added to the fertilizer after treatment.
DE4343569 relates to a base material for the production of compost comprising an inerted or coated base material (selected from the group including but not limited to oat hulls). The inertization process is carried out using the silicification process with the use of silica, water glass and similar reagents, and the coating process is carried out with the use of wax or resin.
European patent application EP3814453 discloses compositions for increasing the immunity, health, growth and productivity of plants using a combination of microorganisms (preferably a combination of *Trichoderma spp.* and *Bacillus spp,* preferably in a ratio of 1:4) and/or by-products of their growth. The composition may advantageously contain glucose, e.g. in the form of molasses. The composition may be in the form of a powder or granules.
EP3569585 discloses the use of oat bran (preferably pelletized/granulated oat hull, preferably in an amount of 5-50%) as the main ingredient or additive in a plant substrate. This document does not describe any process for treating oat hulls prior to granulation.

Despite the progress in the field of bioformulations based on selected microorganisms, there is still a need for products and methods that would enable plant bioprotection, e.g. protection of plants against microbiological pathogens, while not using agrochemicals. Solving these problems was one of the aims of the present invention.

The aim of the invention was primarily to develop a bioformulation based on granular, purified oat hull for use in vegetable cultivation, which would improve the microbiological and physicochemical properties of the soil and increase yielding as well as improve the morphological characteristics of plants. It turned out that a bioformulation containing carrier granulate based on oat hull with the addition of molasses inoculated with microorganisms meets these requirements and therefore provides an attractive alternative to existing mineral preparations and plant protection products.

### The object of the invention

In the first aspect, the object of the invention is the bioformulation defined in claim 1 and dependent claims 2-6, comprising carrier granulate comprising granulated, purified oat hull and molasses, and at least one microbiological additive selected from a group comprising at least one fungus of the genus *Trichoderma* and at least one bacterium selected from the PGPR group, wherein the granulated oat hull has purity between about 98 and about 99,90 % and is free of microbiological contamination. The oat hull obtains this characteristic as a result of purifying the lignocellulosic raw material and optionally by sterilization process of the purified raw material.

In the second aspect, the object of the invention is the method of producing the bioformulation according to the invention, as defined in claims 7-11.

In the third aspect, the object of the invention is the method of distributing the bioformulation to the soil as defined in claim 12.

In the fourth respect, the present invention relates to carrier granulate as defined in claim 13, comprising granulated oat hull and molasses, wherein granulated oat hull has purity between about 98 and about 99,90% and is free of microbiological contamination.

In the fifth aspect of the invention, the present invention relates to the method of production of the said carrier granulate as defined in claims 14-15.

### Advantages of the invention

Granulated oat hull pre-treated by purification and optionally sterilization process and appropriate strains of bacteria from PGPR group, preferably *Bacillus* sp. and *Pseudomonas* sp. and/or fungi of the genus *Trichoderma* sp. additionally inoculated on said hull will enable the introduction of additional easily digestible minerals, vitamins, organic acids, amino acids, sugars and other substances that have a positive effect on plant growth and substances that stimulate the resistance of plants to stress into the soil.

Bacteria and/or fungi introduced onto granulated, purified oat hull thanks to producing plant growth-stimulating substances, including phytohormones, will have the effect of increasing yielding while reducing the use of chemicals.

Furthermore, microorganisms used in the intended fertilizer preparation, besides the properties promoting the growth and development of plants, are species safe for humans and animals and have the GRAS *(Generally Recognized As Safe)* status, and therefore they can be used by a wide range of agricultural and horticultural producers, starting from allotment holders to farmers having large areas of agricultural lands. Among the most common pathogens destroying agricultural crops in the world are fungi of the genus *Fusarium,* as well as *Alternaria,* which cause significant crop losses.

The bioformulation, as a carrier of waste raw materials and the aforementioned microorganisms, will have a positive impact on the soil by accelerating the mineralization processes of organic matter, improving the pH of the soil and increasing water retention in the soil. Bacteria and fungi improve soil structure by secreting mucus of the bacterial capsule, which causes the formation of a spongey, lumpy substrate structure. In addition, through the production of extracellular polysaccharides and other cellular metabolites, microorganisms affect the physical properties of the substrate, helping to stabilize its structure. The developed group of the produced bioformulations, in addition to the positive effect on plants, will further improve habitat conditions for microorganisms, while reducing the number of pathogenic microorganisms, which will result in an increase in the yield of selected plants and improvement of nutritional values in terms of macronutrients and micronutrients.

The bioformulation based on purified granulated oat hull with molasses inoculated with microorganisms according to invention can be used in the cultivation of horticultural plants having economic importance. Depending on the dose size and type of soil, it contributes to reducing the development of pathogenic molds in the soil (see Example 2 and 3) and/or an increase in dehydrogenase activity and thus its fertility (see Examples 4 and 5), as well as positively affects the yield of the tested plants (see Examples 6 and 7) and their chemical composition in terms of macronutrients and micronutrients (see Example 8 and 9).

The reactions of plants and pathogens are varied and depend on the species of cultivated plants and the nutrient content in the soil, as well as the dose of introduced bioformulation. Therefore, depending on the type of plant, it is important to select additives (microorganisms) introduced into carrier granulate (granulated hull with molasses) into a specific soil type (soil having a higher and lower nutrient content). The advantage of using bioformulation according to the invention is the wide spectrum of its action regardless of the type of crop or soil. In addition, it makes possible to increase the yield of vegetable crops, which increases its competitiveness to conventional fertilization. In addition to the agronomic aspect (integrated plant cultivation - Directive 2009/128/EC), bioformulation according to the invention provides an environmentally attractive alternative to the use of mineral fertilizers and chemical plant protection.

The carried out research has shown that the bioformulations according to the invention:
- improve the biological fertility of the soil,
- significantly improve the water availability for plants,
- have a strong phytosanitary effect by limiting the development of pathogenic fungi of the genus *Alternaria* and *Fusarium,*
- improve the plants immunity to stress factors, e.g. drought,
- improve the nutrition of plants, resulting in an increase of the content of valuable protein and macronutrients Ca, Mg, K and P,
- increase crop yield,
- trigger the mobilisation of mineral forms of phosphorus in the soil,
- induce the systemic immunity of plants, and
- contribute to an increase in the absorbent surface area of the roots

All the advantages described above result, on the one hand, from the qualitative and quantitative composition of the bioformulation according to the invention and, on the other hand, from the pre-treatment process of oat hull before it is processed into carrier granulate.

### Detailed description of the invention

The bioformulation according to the invention is based on carrier granulate produced with pre-treated, granulated oat hull and molasses.

### Carrier granulate

The starting material for the production of granular oat hull is a lignocellulosic raw material derived from oat threshing, containing, among others, oat hulls, straw, chaff, weeds, seeds of other plants, ears and the like. Before the granulation process, this material must undergo a pre-treatment including the following processes:
- purification of the lignocellulosic raw material by removing straw, chaff, weeds, seeds of other plants, chaff, ears, to obtain a fraction containing from about 98 to about 99.9% of oat hulls, and
- optionally sterilization of obtained fraction of oat hulls to remove microbiological contaminations

The purification step of lignocellulosic raw material can be carried out by any technique known to the person skilled in the art, wherein preferably it is carried out using a pneumatic separator with the function of calibration of separated material.

The purification step of lignocellulosic raw material is used to remove "foreign" materials that could disrupt the granulation process, the process of inoculation of carrier granulate with microbiological components, as well as could affect the efficiency of the bioformulation.

At the end of the purification step, a fraction comprising about 98 to about 99,9 %, preferably from about 98,5 to about 99,9 %, even more preferably from about 99 to about 99,9 % of the oat hulls, is obtained. The content of oat hulls in the said fraction is identical with /determines the purity of this fraction. Specifically, if said fraction contains 98% of the oat hull, it means that it is 98% pure.

The sterilization step can similarly be carried out by any technique known to the person skilled in the art, which leads to the essential removal of microbiological contamination from the oat hull. The term essentially means that at least 90%, preferably 95%, more preferably 96%, even more preferably at 97%, particularly preferably 98%, for example 99%, even more preferably at 99.9% and most preferably at 100% of said microbiological contamination is removed.

In the preferred embodiment of the invention, the sterilization step is carried out by the use of dry steam.

In one of the preferred embodiments of the invention, pre-treatment includes only the purification step of lignocellulosic raw material. In another preferred embodiment of invention, the pre-treatment of lignocellulosic raw material includes both the purification stage and the sterilization stage.

After pre-treatment, the obtained oat hulls fraction is mixed with molasses in appropriate proportions and then the resulting mixture is granulated.

According to the invention, any molasses used in the art may be used to prepare said mixture, preferably the molasses is selected from the group comprising beet molasses and sugar cane molasses. Most preferably, beet molasses is used according to the present invention.

Preferably, the mixture is prepared using oat hull fraction containing fine porous particles. Preferably, the average particle size is not more than 4 mm.

The process of granulating the mixture of oat hulls and molasses may be carried out by any granulation/pelleting technique known to the person skilled in the art, preferably it is carried out by forming the granulate in an annular granulator with the horizontally working matrix.

In a preferred embodiment of the invention, the carrier granulate comprises granulated oat hull and molasses. In another preferred embodiment of the invention, the carrier granulate consists of granulated oat hull and molasses.

The purified oat hull constitutes from about 97 to about 99% by weight, based on the weight of the carrier granulate, and the molasses constitutes from 1 to about 3% by weight, based on the weight of the carrier granulate.

### Bioformulation

The carrier granulate described above serves as a substrate carrying the microbiological additives.

The carrier granulate constitutes from about 98 to 99% by weight in relation to the weight of the bioformulation, wherein the granulated oat hull constitutes from about 95 to about 98% by the weight of the bioformulation, molasses constitutes from about 1 to about 3% by the weight of the bioformulation, and the microbiological additives constitute from about 1 to about 2% by weight of the bioformulation.

According to the invention, the microbiological additives include at least one fungus of the genus *Trichoderma* and/or at least one bacterium selected from the PGPR group.

The fungus of the genus *Trichoderma* may be any known fungus belonging to this genus, which can advantageously be used in bioformulations having purposes the same as the bioformulation of the present invention, and therefore including agricultural or horticultural use, preferably as plant growth promoter, phytosanitary formulation, plant protection product, soil conditioner or biostimulant. In a particularly preferred embodiment of the invention, the fungus of the genus *Trichoderma* is a fungus of the species *Trichoderma asperellum,* for example *Trichoderma asperellum* or *Trichoderma asperellum Eko.*

The fungus of the genus *Trichoderma* may be present in the bioformulation in amount of about 1 to about 2 % by weight of the bioformulation.

The bacteria of the PGPR (plant growth promoting rhizobacteria) group are bacteria that exhibit a beneficial impact on plants. The most numerous representatives of PGPR belong to the genera *Bacillus, Pseudomonas* and *Azospirillum.* These microorganisms can have a positive effect on plants, mainly by direct and/or indirect stimulation of their growth. According to the generally available knowledge in the field of the invention, PGPR bacteria can be treated as biological fertilizing agents, promoting plant growth, colonizing the soil thanks to the ability to adapt to various environmental conditions, fast growth rate and metabolize many different substrates, directly and indirectly influencing growth, development and yielding of arable crops.

According to the invention, the bioformulation may comprise at least one of the bacteria belonging to PGPR, and preferably it contains at least one bacterium of the genus *Bacillus* and/or at least one bacterium of the genus *Pseudomonas.* In one of the preferred embodiments of the invention, the bioformulation comprises only at least one bacterium of the genus *Bacillus.* In another preferred embodiment of the invention, the bioformulation comprises only at least one bacterium of the genus *Pseudomonas.* In yet another preferred embodiment of the invention, the bioformulation comprises both at least one bacterium of the genus *Pseudomonas* and at least one bacterium of the genus *Bacillus.* In a particularly preferred embodiment of the invention, the bacterium of the genus *Pseudomonas* is a bacterium of the species *Pseudomonas fluorescens.* In a particularly preferred embodiment of the invention, the bacterium of the genus *Bacillus* is a bacterium of the genus *Bacillus subtilis.*

At least one bacterium of the PGPR group may be present in amount of about 1% do 2% by weight of the bioformulation, which constitutes 10⁴ CFU/g of preparation.

In one preferred embodiment of the invention, the bioformulation contains only fungus of the species *Trichoderma asperellum.* In another particularly preferred embodiment of the invention, the bioformulation comprises a fungus of the species *Trichoderma asperellum* and a bacterium of the species *Bacillus subtilis.* In a further particularly preferred embodiment of the invention, the bioformulation comprises a fungus of the species *Trichoderma asperellum* and a bacterium of the species *Pseudomonas fluorescens.* In another particularly preferred embodiment of the invention, the bioformulation comprises a fungus of the species *Trichoderma asperellum,* a bacterium of the species *Bacillus subtilis* and a bacterium of the species *Pseudomonas fluorescens.*

The microbiological additives can be prepared for application on the carrier granulate by any method known to the person skilled in the art. However, preferably the organic additives are prepared by:
- preparing a suspension of at least one microbiological additive selected from the group comprising at least one fungus of the genus *Trichoderma* and/or at least one bacterium of the PGPR group,
- incubation of the obtained suspension.

In said suspension, the microorganisms are preferably proliferated on selective culture media.

Preferably, the incubation of the suspension is carried out depending on the kind of microorganisms from about 72 hours to about 12-14 days on a shaker for bacteria and for about 12 do about 12 days for fungi of genus *Trichoderma.*

The thus prepared suspension of microbiological additives is applied to the carrier granulate by any known technique, preferably by spraying or atomization. Advantageously, this leads to an inoculation of microbiological additives in the pores of the granulated oat hull. In a particularly preferred embodiment of the invention, the application of the microbiological additives is followed by incubation and drying of the carrier granulate coated with said suspension.

### Use of the invention

The bioformulation according to the invention is used in agriculture and horticulture, inter alia, as a plant growth agent, phytosanitary formulation, plant protection agent, soil conditioner or biostimulant.

Advantageously, the bioformulation can be applied by any known technique. The amount of the applied bioformulation can be determined by the person skilled in the art based on the data presented in the subject description.

In a particularly preferred embodiment of the invention, the bioformulation according to the invention can be used as a soil biostimulant, which is supplied using sidedress application in an amount of 400 to 800 kg/ha by means of a point seeder during the sowing seeds into the soil, by placing the fertilizer granulate at a distance not greater than 5 cm below the seed line or to the side of the seed row.

The carrier granulate according to the invention is used in analogous technical fields as a carrier for active ingredients other than those indicated for the bioformulation according to the invention, which have a beneficial effect on the soil and/or plants. As shown below, the carrier granulate shows very good properties in terms of delivering the active ingredients (microbiological additives) to the soil and/or plants.

According to the invention, the term "about" used above and below should be understood as a deviation of +/- 5% from the given value, reflecting inaccuracies that may appear during the process of producing the bioformulation and carrier granulate according to the invention, e.g. during measuring ingredients

### Examples

The following examples are provided only to illustrate the invention and to explain its various aspects, without limiting it, and should not be read as a full scope thereof, which is defined in the appended claims. In the following examples, used unless otherwise indicated, standard materials and methods used in the art or the manufacturer's recommendations for specific reagents and methods were used.

The bulk density of the oat hull substrate used in the production of the granulate was 206.00 g/l with a moisture content of 14.50 %. The following materials were used for the tests: bacterial strains *Bacillus subtilis, Pseudomonas fluorescens* and molds *Trichoderma aseprellum.*

The analysis of the effectiveness of the bioformulation was carried out in a greenhouse adapted to carry out a full process with provision of appropriate conditions, in pots of various types and sizes, to perform many experimental series in a short time. The possibilities of using the new bioformulation in the cultivation of various horticultural plants were assessed:
- by the use of various doses of the biostimulant (lower dose of 8 g/plant and higher dose of 20g/plant),
- method of application (near seeds),
- on different types of soil, differentiated in terms of the content of organic matter (I - soil having a higher content of organic matter - 4.85%, II - soil having a lower content of organic matter - 2.56%)

A wide spectrum of research in greenhouse conditions was used to select the best bioformulations and to formulate recommendations for the application of the bioformulation for users.

The selected bioformulations for broadcast application on the soil having a higher content of substances, dedicated to selected plants include: WMT, WMTP, WMTB, WMTEko. On the soil having a lower content of organic matter, the broadcast application of such bioformulations as: WMT, WMTEko and WMTP was the best.

### Example 1: Method of production of bioformulations

### Preparation of carrier granulate,

The carrier granulate consisting of:
a) oat hull, and
b) beet molasses,
was produced.

The lignocellulosic raw material derived from oat threshing, constituting a mixture of straw, weeds, seeds of other plants, chaff, ears, hulls and the like was pretreated, by:
- purification by removing of straw, chaff, weeds, seeds of other plants, ears, resulting in obtaining of fraction containing 98% oat hulls,
- sterilization of obtained fraction of oat hulls to remove microbiological contamination

The carrier granulate ingredients (oat hulls and beet molasses) were combined and mixed for 20 min, and then pelletized in a granulator equipped with a matrix with a diameter of 4.8 mm. The granulate was then cooled to ambient temperature in a rotary cooler. In the final mass of the carrier granulate, the molasses content is from 1% to 3%, and the oat hull content is from 97 to 99%.

### Preparation of Bacillus subtilis, Pseudomonas fluorescens bacteria and molds of genus Trichoderma inoculum for carrier granulate inoculation

For this purpose, a suspension of microorganisms was prepared, which had been grown with the selective culture medium; broth medium for the proliferation of bacteria or a solution of sugar cane molasses (5%) for the proliferation of fungi.

Before the proliferation of the microorganisms in the liquid broth substrate or molasses solution with which the pellet was inoculated by spraying, cultures were carried out on solid selective media in Petri dishes. This procedure was necessary in order to trigger metabolic activation of lyophilized microorganisms. Bacteria of the genus *Bacillus* and *Pseudomonas* were grown on Nutrient Agar with yeast extract, Aminobak and NaCl; fungi on PDA (Potato Dextrose Agar) substrate -. After subculturing the indicated microorganisms three times, the Petri dishes were flooded with 5 ml of saline, and then the homogeneous suspension obtained by vortexing on a Vortex device was sterile transferred to a liquid broth or molasses solution. After 72 hours of incubation of bacterial culture on a shaker and 12-14 days of incubation of fungi without the use of a shaker, a concentration of bacteria cells/conidial spores of fungi was achieved at the level of 10⁶-10⁷/1 ml of medium. With the thus prepared microorganism suspension, the carrier granulate was inoculated in a ratio of 1:100, i.e. 1 ml of suspension per 100 g of granulate.

Introduction by spraying the suspensions obtained in the previous stage onto the carrier granules in the amount of 1 ml of suspension per 100 g of pellet (granulate).

After incubation and drying of the obtained bioformulation (in order to inhibit the metabolism of bacteria), it was packed and stored at 15 °C. The high absorbency of the lignocellulosic substrate guarantees good adhesion of the microorganism suspensions obtained in the previous step to the product surface.

According to the above method, the following variants of bioformulations were obtained:

**Bioformulation WMT**

| | |
|---|---|
| about 96% by weight | Oat hull |
| about 3% by weight | Molasses |
| about 1% by weight | *Trichoderma asperellum* (T) |

**Bioformulation WMTEko -**

| | |
|---|---|
| about 96% by weight | Oat hull |
| about 3% by weight | Molasses |
| about 1% by weight | *Trichoderma asperellum* (TEko) |

**Bioformulation WMTP**

| | |
|---|---|
| about 95% by weight | Oat hull |
| about 3% by weight | Molasses |
| about 1% by weight | *Trichoderma asperellum* (T) |
| about % by weight | *Pseudomonas fluorescens* |

**Bioformulation WMTB**

| | |
|---|---|
| about 95% by weight | Oat hull |
| about 3% by weight | Molasses |
| about 1% by weight | *Trichoderma asperellum* (T) |
| about 1% by weight | *Bacillus subtilis* |

**Example 2.** Analysis of the effectiveness of bioformulations, granulates based on oat hull with applied microorganisms, broadcast applied, against fungal pathogens, on soil having a higher content of organic matter, depending on the dose, under controlled conditions for selected plants (pot experiment).

**Table 1: Analysis of the effectiveness of bioformulations on soil having a higher content of organic matter according to the invention in pot experiments.**

| Plant | count of *Fusarium* sp. *10² CFU/g dm soil | | count of *Alternaria* sp. *10² jtk/g dm soil | |
|---|---|---|---|---|
| | **Bioformulation WMT- lower dose** | | | |
| beetroot | "0" | WMT | "0" | WMT |
| | 10.76 | 5.68 | 10.43 | 3.55 |
| Chinese cabbage | "0" | WMT | "0" | WMT |
| | 8.0 | 4.3 | 9.43 | 3.88 |
| pepper | "0" | WMT | "0" | WMT |
| | 25.8 | 12.3 | 2.1 | 0.38 |
| carrot | "0" | WMT | "0" | WMT |
| | 12.93 | 4.57 | 6.1 | 0.36 |

| Plants | **Bioformulation WMTP - higher dose** | | | |
|---|---|---|---|---|
| beetroot | "0" | WMTP | "0" | WMTP |
| | 12.14 | 6.75 | 1.31 | 0.77 |
| cabbage | "0" | WMTP | "0" | WMTP |
| | 13.28 | 7.14 | 1.77 | 0.59 |

| Plants | **Bioformulation WMTEko - higher dose** | | | |
|---|---|---|---|---|
| carrot | "0" | WMTEko | "0" | WMTEko |
| | 9.38 | 6.87 | 6.23 | 3.16* |
| radish | "0" | WMTEko | "0" | WMTEko |
| | 9.07 | 7.40 | 1.08 | 0.39 |

| Plants | **Bioformulation WMTB - higher dose** | | | |
|---|---|---|---|---|
| cabbage | "0" | WMTB | "0" | WMTB |
| | 13.27 | 6.00 | 1.79 | 0.20 |

| | | | | |
|---|---|---|---|---|
| "0-" conventional fertilization * lower dose of bioformulation | | | | |

### Conclusions

Following effects were observed:
- the stimulating effect of the bioformulation produced based on granulated oat hull with the addition of molasses and the applied fungi of the genus *Trichoderma* (WMT) used at a lower dose on the phytosanitary properties of the soil having a higher content of substances resulting from the restriction of the growth of pathogenic fungi of the genus *Fusarium* and *Alternaria* in the cultivation of: beetroot, Chinese cabbage, carrot and pepper,
- the stimulating effect of the bioformulation produced based on granulated oat hull with the addition of molasses and the applied fungi of the genus *Trichoderma* and bacteria *Pseudomonas fluorescens* (WMTP) used at a higher dose on the phytosanitary properties of the soil having a higher content of substances resulting from the restriction of the growth of pathogenic fungi of the genus *Fusarium* and *Alternaria* in the cultivation of: beetroot and cabbage,
- the stimulating effect of the bioformulation produced based on granulated oat hull with the addition of molasses and the applied fungi of the genus *Trichoderma* (WMTEko), applied at a higher dose on the phytosanitary properties of the soil having a higher content of organic substance resulting from the restriction of the growth of pathogenic fungi of the genus *Fusarium* and *Alternaria* in the cultivation of: carrot and radish,
- the stimulating effect of the bioformulation produced based on granulated oat hull with the addition of molasses and the applied fungi of the genus *Trichoderma* and bacteria *Bacillus subtilis* (WMTB), applied at a higher dose on the phytosanitary properties of the soil having a higher content of organic substance resulting from the restriction of the growth of pathogenic fungi of the genus *Fusarium* and *Alternaria* in the cultivation of: cabbage.

**Example 3.** Analysis of the effectiveness of bioformulations, granulates based on oat hull with applied microorganisms, broadcast applied, against fungal pathogens, on soil having a lower content of organic matter, depending on the dose, under controlled conditions for selected plants (pot experiment).

**Table 2: Analysis of the effectiveness of bioformulations on soil having a lower content of organic matter according to the invention in pot experiments.**

| Plant | Count of *Fusarium* sp. *10² CFU/g dm soil | | Count of *Alternaria* sp. *10² CFU/g dm soil | |
|---|---|---|---|---|
| | **Bioformulation WMT - higher dose** | | | |
| beetroot | "0" | WMT | "0" | WMT |
| | 22.45 | 18.59 | 3.75 | 4.07 |
| Chinese cabbage | "0" | WMT | "0" | WMT |
| | 14.68 | 7.92 | 0.97 | 0.79* |

| | **Granulate WMT with applied fungi of the genus *Trichodrema -* lower dose** | | | |
|---|---|---|---|---|
| carrot | "0" | WMT | "0" | WMT |
| | 9.94 | 7.89 | 1.68 | 0.74* |

| Plants | **Bioformulation WMTP - higher dose** | | | |
|---|---|---|---|---|
| | "0" | WMTP | "0" | WMTP |
| Chinese cabbage | 14.68 | 11.13 | 0.98 | 0.19 |
| radish | "0" | WMTP | "0" | WMTP |
| | 8.56 | 6.95 | 2.31 | 0.57 |

| | **Bioformulation WMTEko - higher dose** | | | |
|---|---|---|---|---|
| carrot | "0" | WMTEko | "0" | WMTEko |
| | 9.95 | 5.27 | 1.68 | 0.54* |
| radish | "0" | WMTEko | "0" | WMTEko |
| | 8.56 | 4.65 | 2.31 | 0.38 |

### Conclusions

Following effects were observed:
- the stimulating effect of the bioformulation produced based on granulated oat hull with the addition of molasses and the applied fungi of the genus *Trichoderma* (WMT) on the phytosanitary properties of the soil having a lower content of organic substance resulting from the restriction of the growth of pathogenic fungi of the genus *Fusarium* and *Alternaria* in the cultivation of: beetroot, Chinese cabbage at a higher dose and carrot at a lower dose,
- the stimulating effect of the bioformulation produced based on granulated oat hull inoculated with fungi of the genus *Trichoderma* and bacteria *Pseudomonas fluorescens* (WMTP) applied at the higher dose on the phytosanitary properties of the soil having a lower content of organic substance resulting from the restriction of the growth of pathogenic fungi of the genus *Fusarium* and *Alternaria* in the cultivation of: Chinese cabbage and radish,
- the stimulating effect of the bioformulation produced based on granulated oat hull with the addition of molasses and the applied fungi of the genus *Trichoderma* (WMTEko) applied at the higher dose on the phytosanitary properties of the soil having a lower content of organic substance resulting from the restriction of the growth of pathogenic fungi of the genus *Fusarium* and *Alternaria* in the cultivation of: carrot and radish.

**Example 4.** Analysis of the effectiveness of bioformulations, granulate based on oat hull with applied microorganisms, on soil having a lower content of organic matter, with regard to dehydrogenases activity and soil fertility, depending on the dose, under controlled conditions for selected plants (pot experiment).

**Table 3: Analysis of the effectiveness of bioformulations according to the invention on soil having a higher content of organic matter in pot experiments.**

| Plant | Level of dehydrogenases activity | | Level of soil fertility BIF | |
|---|---|---|---|---|
| | **Bioformulation WMT- lower dose** | | | |
| **beetroot** | "0" | WMT | "0" | WMT |
| | 0.042 | 0.05 | 9.5 | 11.50 |
| **Chinese cabbage** | "0" | WMT | "0" | WMT |
| | 0.012 | 0.023 | 28.72 | 28.23 |
| **pepper** | "0" | WMT | "0" | WMT |
| | 0.015 | 0.052 | 3.34 | 11.80 |
| **carrot** | "0" | WMT | "0" | WMT |
| | 0.038 | 0.083 | 8.61 | 18.81 |

| Plants | **Bioformulation WMTP - higher dose** | | | |
|---|---|---|---|---|
| beetroot | "0" | WMTP | "0" | WMTP |
| | 0.042 | 0.055 | 9.54 | 12.37 |
| cabbage | "0" | WMTP | "0" | WMTP |
| | 0.013 | 0.027 | 2.88 | 6.05 |

| Plants | **Bioformulation WMTEko - higher dose** | | | |
|---|---|---|---|---|
| carrot | "0" | WMTEko | "0" | WMTEko |
| | 0.038 | 0.075 | 8.60 | 17.05 |
| Radish | "0" | WMTEko | "0" | WMTEko |
| | 0.025 | 0.044 | 5.68 | 9.89 |

| Plants | **Bioformulation WMTB - higher dose** | | | |
|---|---|---|---|---|
| cabbage | "0" | WMTB | "0" | WMTB |
| | 0.012 | 0.023 | 2.88 | 5.11 |

### Conclusions

- Following effects were observed: the stimulating effect of the bioformulation produced based on granulated oat hull with the addition of molasses and the applied fungi of the genus *Trichoderma* (WMT), applied at a lower dose on the biochemical properties of soil having a higher content of organic matter, resulting from an increase in the level of dehydrogenases activity in the soil and the BIF index in cultivation of: beetroot, Chinese cabbage, carrot and pepper,
- the stimulating effect of the bioformulation produced based on granulated oat hull with the addition of molasses inoculated with fungi of the genus *Trichoderma* and bacteria *Pseudomonas fluorescens* (WMTP), applied at a higher dose on the biochemical properties of soil having a higher content of organic matter, resulting from the increase in the level of dehydrogenases activity in the soil and BIF indicator, in the cultivation of: beetroot and cabbage,
- the stimulating effect of the bioformulation produced based on granulated oat hull with the addition of molasses and applied fungi of the genus *Trichoderma* (WMTEko), applied in a higher dose on the biochemical properties of soil having a higher content of organic matter, resulting from an increase in the level of dehydrogenases activity in the soil and the BIF index in the cultivation : carrot and radish
- stimulating effect of the bioformulation produced based on granulated oat hulls and the applied fungi of the genus *Trichoderma* and bacteria Bacillus subtilis (WMTB), applied in a higher dose on the biochemical properties of the soil, with a higher content of organic matter, resulting from an increase in the level of dehydrogenase activity in the soil and the BIF index in cultivation of: cabbage.

**Example 5:** Analysis of the effectiveness of bioformulations, granulates based on oat hull with applied microorganisms, broadcast applied on soil having a lower content of organic matter, with regard to dehydrogenases activity and soil fertility, depending on the dose, under controlled conditions for selected plants (pot experiment).

**Table 4: Analysis of the effectiveness of bioformulations according to the invention on soil having a lower content of organic matter in pot experiments.**

| Plant | Level of dehydrogenases activity | | Level of soil fertility BIF | |
|---|---|---|---|---|
| | **Bioformulation WMT- higher dose** | | | |
| beetroot | "0" | WMT | "0" | WMT |
| | 0.005 | 0.032 | 1.16 | 7.34 |
| Chinese cabbage | "0" | WMT | "0" | WMT |
| | 0.006 | 0.058 | 1.38 | 13.14 |

| | **Bioformulation WMT - lower dose** | | | |
|---|---|---|---|---|
| **carrot** | "0" | WMT | "0" | WMT |
| | 0.019 | 0.047 | 4.44 | 10.68 |

| Plants | **Bioformulation WMTP- higher dose** | | | |
|---|---|---|---|---|
| **Chinese cabbage** | "0" | WMTP | "0" | WMTP |
| | 0.006 | 0.013 | 1.39 | 3.03 |
| **Carrot** | "0" | WMTP | "0" | WMTP |
| | 0.019 | 0.063 | 4.44 | 14.23 |
| **Radish** | "0" | WMTP | "0" | WMTP |
| | 0.025 | 0.055 | 5.68 | 12.52 |

| | **Bioformulation WMTEko- higher dose** | | | |
|---|---|---|---|---|
| Carrot | "0" | WMTEko | "0" | WMTEko |
| | 0.019 | 0.060 | 4.44 | 13.69 |
| Radish | "0" | WMTEko | "0" | WMTEko |
| | 0.025 | 0.044 | 5.68 | 9.89 |

### Conclusions:

Following effects were observed:
- the stimulating effect of the bioformulation produced based on granulated oat hulls with the addition of molasses and the applied fungi of the genus *Trichoderma* (WMT), on the biochemical properties of soil having a lower content of organic matter, resulting from an increase in the level of dehydrogenase activity in the soil and the BIF index in cultivation : beetroot, Chinese cabbage at higher dose and carrot at lower dose,
- the stimulating effect of the bioformulation produced based on granulated oat hull with the addition of molasses inoculated with fungi of the genus *Trichoderma* and bacteria *Pseudomonas fluorescens* (WMTP), applied in a higher dose on the biochemical properties of soil having a higher content of organic matter, resulting from the increase in the level of dehydrogenases activity in the soil and BIF indicator, in the cultivation of: Chinese cabbage and radish,
- the stimulating effect of the bioformulation produced based on granulated oat hull with the addition of molasses and the applied fungi of the genus *Trichoderma* (WMTEko) applied at the higher dose on the biochemical properties of the soil having a lower content of organic matter, resulting from the increase in the level of dehydrogenase activity in the soil and BIF indicator, in the cultivation of: carrot and radish.

**Example 6:** Analysis of the effectiveness of bioformulations, granulates based on oat hull with applied microorganisms, broadcast applied, with regard to yielding of plants on soil having a higher content of organic matter, depending on the dose, under controlled conditions for selected plants (pot experiment).

**Table 5: Analysis of the effectiveness of bioformulations according to the invention on soil having a higher content of organic matter in pot experiments**

| Plant | Yield of fresh plant mass (seeds) [g / plant] | |
|---|---|---|
| | Yield of seeds (t/ha)* | |
| | Yield of roots (t/ha)** | |
| | **Bioformulation WMT- lower dose** | |
| **beetroot** | "0" | WMT |
| | 3.10 | 4.49 |
| **Chinese cabbage** | "0" | WMT |
| | 26.81 | 32.185 |
| **pepper** | "0" | WMT |
| | 513.15 | 910.43 |
| **carrot** | "0" | WMT |
| | 154.26 | 159.99 |

| Plants | **Bioformulation WMTP** - **higher dose** | |
|---|---|---|
| Beetroot | "0" | WMTP |
| | 33.6 | 41.64 |
| Cauliflower | "0" | WMTP |
| | 70.81 | 82.15 |

| Plants | **Bioformulation WMTEko - lower dose** | |
|---|---|---|
| Carrot | "0" | WMTEko |
| | 133.37 | 137.06 |
| Radish | "0" | WMTEko |
| | 2.09 | 2.58 |

| Plants | **Bioformulation WMTB - higher dose** | |
|---|---|---|
| Chinese cabbage | "0" | WMTB |
| | 26.81 | 40.69 |

| | | |
|---|---|---|
| "0 - "conventional fertilization * lower dose of the bioformulation | | |

### Conclusions

Following effects were observed:
- the stimulating effect of the bioformulation produced based on granulated oat hull and the addition of molasses and the applied fungi of the genus *Trichoderma* (WMT), applied at a lower dose, on the yielding of plants on soil having a higher organic substance content, in the cultivation of: beetroot, Chinese cabbage, carrot and pepper,
- the stimulating effect of the bioformulation produced based on granulated oat hulls with the addition of molasses inoculated with fungi of the genus *Trichoderma* and bacteria *Pseudomonas fluorescens* (WMTP), applied at a higher dose on the yielding of plants on soil having a higher content of organic matter, in the cultivation of: beetroot and cauliflower,

- the stimulating effect of the bioformulation produced based on granulated oat hull with the addition of molasses and the applied fungi of the genus *Trichoderma* (WMTEko) applied at the lower dose on the yielding of plants on soil having a higher content of organic matter, in the cultivation of: carrot and radish,
- the stimulating effect of the bioformulation produced based on granulated oat hull with the addition of molasses inoculated with fungi of the genus *Trichoderma* and bacteria *Bacillus subtilis* (WMTB), applied in a higher dose on the yielding of plants on soil having a higher content of organic matter, in the cultivation of: Chinese cabbage.

**Example 7:** Analysis of the effectiveness of bioformulations, granulates based on oat hull with applied microorganisms, broadcast applied, with regard to yielding of plants on soil having a lower content of organic matter, depending on the dose, under controlled conditions for selected plants (pot experiment).

**Table 6: Analysis of the effectiveness of bioformulations according to the invention on soil having a lower content of organic matter in pot experiments**

| Plant | Yield of fresh plant mass (seeds) [g/plant] | |
|---|---|---|
| | Yield of seeds (t/ha)* | |
| | Yield of roots (t/ha)** | |
| | **Bioformulation WMT - lower dose** | |
| **beetroot** | "0" | WMT |
| | 23.38 | 27.64 |
| **Chinese cabbage** | "0" | WMT |
| | 20.33 | 21.83 |
| **Carrot** | "0" | WMT |
| | 114.85 | 126.32 |

| Plant | **Bioformulation WMTP - higher/lower dose*** | |
|---|---|---|
| Chinese cabbage | "0" | WMTP |
| | 26.81 | 29.13* |
| Carrot | "0" | WMTP |
| | 114.85 | 139.63/119.78* |
| Radish | "0" | WMTP |
| | 2.09 | 2.41/2.38* |

| | **Bioformulation WMTEko - higher dose** | |
|---|---|---|
| Carrot | "0" | WMTEko |
| | 114.85 | 140.43/121.76* |
| Radish | "0" | WMTEko |
| | 2.09 | 2.44/2.58* |

| | | |
|---|---|---|
| *lower dose | | |

### Conclusions:

Following effects were observed:
- the stimulating effect of the bioformulation produced based on granulated oat hull and the addition of molasses and the applied fungi of the genus *Trichoderma* (WMT), at the lower dose on the yielding of plants on soil having a lower organic substance content, in the cultivation of: beetroot, Chinese cabbage, carrot and pepper,
- the stimulating effect of the bioformulation produced based on granulated oat hull with the addition of molasses inoculated with fungi of the genus *Trichoderma* and bacteria *Pseudomonas fluorescens* (WMTP), applied at the higher dose on the yielding of plants on soil having a lower content of organic matter, in the cultivation of: Chinese cabbage, carrot and radish, however the increase of yielding was also observed at the lower dose,
- the stimulating effect of the bioformulation produced based on granulated oat hull with the addition of molasses and the applied fungi of the genus *Trichoderma* (WMTEko) applied at the higher dose on the yielding of plants on soil having a lower content of organic matter, in the cultivation of: carrot and radish.

**Example 8:** Analysis of the effectiveness of bioformulations, granulates based on oat hull with applied microorganisms, broadcast applied, with regard to protein and macronutrients (Ca, K and/or Mg and P) level in plants on soil having a higher content of organic matter, depending on the dose, under controlled conditions for selected plants (pot experiment).

**Table 7: Analysis of the effectiveness of bioformulations according to the invention on soil having a higher content of organic matter in pot experiments**

| **Plants** | **Protein level %** | | **Ca ppm** | | **Mg or P* ppm** | | **K ppm** | | |
|---|---|---|---|---|---|---|---|---|---|
| | **Bioformulation WMT- lower dose** | | | | | | | | |
| | "0" | WMT | "0" | WMT | "0" | WMT | | "0" | WMT |
| lettuce | 10.67 | 11.27 | 14150.79 | 14397.96 | 2430.57 | 2754.30* | | 63770.28 | 64292.22 |
| cabbage | 18.01 | 20.06 | 52799.89 | 58611.68 | 0.000 | 2679.99 | | 31149.32 | 38552.66 |
| cauliflower | 27.77 | 28.99 | 2096.19 | 1577.85 | 8386.42 | 9224.55 | | 68489.18 | 77357.65 |
| carrot | 3.66 | 4.99 | 7501.66 | 7341.46 | 0.00 | 2390.15 | | 42242.37 | 46451.49 |

| Plants | **Bioformulation WMTP - higher dose** | | | | | | | | |
|---|---|---|---|---|---|---|---|---|---|
| | "0" | WMTP | "0" | WMTP | "0" | WMTP | "0" | WMTP | |
| Lettuce | 10.67 | 12.07 | 14150.79 | 15458.65 | 2430.57 | 3022.79* | 63770.28 | 65219.75 | |
| Cabbage | 18.01 | 18.67 | 52799.89 | 79624.53 | 0.000 | 2907.96 | 86318.58 | 77684.15 | |

| Plants | **Bioformulation WMTEko - higher dose** | | | | | | | | |
|---|---|---|---|---|---|---|---|---|---|
| | "0" | WMTEko | "0" | WMTEko | "0" | WMTEko | "0" | WMTEko | |
| Carrot | 3.66 | 6.41 | 7501.66 | 8437.10 | 0.000 | 942.01 | 42242.37 | 51855.02 | |

| Plants | **Bioformulation WMTB - higher dose** | | | | | | | | |
|---|---|---|---|---|---|---|---|---|---|
| | | | | | | | | | |
| Cabbage | 18.01 | 19.79 | 52799.89 | 79624.53 | 0.000 | 2673.82 | 86318.58 | 81315.37 | |

| | | | | | | | | | |
|---|---|---|---|---|---|---|---|---|---|
| *) phosphorus level in soil was provided | | | | | | | | | |

Conclusions

Following effects were observed:
- the stimulating effect of the bioformulation produced based on granulated oat hull and the addition of molasses and the applied fungi of the genus *Trichoderma* (WMT), applied at the lower dose on protein and macronutrients (Ca, K and/or Mg and P) level in plants: lettuce, cabbage, cauliflower and carrot grown in soil having higher organic matter content,
- the stimulating effect of the bioformulation produced based on granulated oat hull with the addition of molasses inoculated with fungi of the genus *Trichoderma* and bacteria *Pseudomonas fluorescens* (WMTP), applied at the higher dose on protein and macronutrients (Ca, K and/or Mg and P) level in plants: lettuce and cabbage, grown in soil having higher organic matter content,
- the stimulating effect of the bioformulation produced based on granulated oat hull with the addition of molasses and the applied fungi of the genus *Trichoderma* (WMTEko) applied at the higher and lower dose on protein and macronutrients (Ca, K and/or Mg and P) level in plant carrot, grown in soil having higher organic matter content,
- the stimulating effect of the bioformulation produced based on granulated oat hull with the addition of molasses and the applied fungi of the genus *Trichoderma* and bacteria *Bacillus subtilis* (WMTB), applied at the higher dose on protein and macronutrients (Ca, K and/or Mg) level in plant - cabbage, in soil having higher organic matter content.

**Example 9:** Analysis of the effectiveness of bioformulations, granulates based on oat hull with applied microorganisms, broadcast applied, with regard to protein and macronutrients (Ca, K and/or Mg and P) level in plants on soil having a lower content of organic matter, depending on the dose, under controlled conditions for selected plants (pot experiment).

**Table 8: Analysis of the effectiveness of bioformulations according to the invention on soil having a lower content of organic matter in pot experiments**

| **Plants** | **Protein level %** | | **Ca ppm** | | **Mg or P* ppm** | | **K ppm** | | |
|---|---|---|---|---|---|---|---|---|---|
| | **Bioformulation WMT - higher dose** | | | | | | | | |
| | "0" | WMT | "0" | WMT | "0" | WMT | | "0" | WMT |
| Lettuce | 6.32 | 8.99 | 16206.39 | 16331.47 | 0.000 | 2095.47 | | 39924.93 | 52517.92 |
| Chinese cabbage | 17.92 | 18.66 | 25827.92 | 20626.54 | 0.000 | 1192.38 | | 31878.54 | 37505.74 |
| Green Beans | 15.23 | 16.72 | 10910.36 | 12114.96 | 3132.95 | 3234.13 | | 38867.97 | 44570.94 |
| carrot | 3.05 | 3.65 | 7826.11 | 8080.28 | 3440.81 | 3835.77 | | 49456.62 | 48685.71 |

| Plants | **Bioformulation WMTP - higher dose** | | | | | | | | |
|---|---|---|---|---|---|---|---|---|---|
| | "0" | WMTP | "0" | WMTP | "0" | WMTP | | "0" | WMTP |
| Lettuce | 6.32 | 9.63 | 16206.39 | 17985.91 | 1681.19 | 2827.81* | 39924.93 | 59626.66 | |
| Cabbage | -not determin ed | - not determin ed | 45222.40 | 62574.5 | 2401.24 | 2665.17 | 82918.62 | 79611.96 | |

| Plants | **Bioformulation WMTEko - higher dose** | | | | | | | | |
|---|---|---|---|---|---|---|---|---|---|
| | "0" | WMTEko | "0" | WMTEko | "0" | WMTEko | "0" | WMTEko | |
| Carrot | 3.05 | 3.59 | 7826.11 | 8887.67 | 2301.92 | 2788.71 | 49456.62 | 37063.88 | |

| | | | | | | | | | |
|---|---|---|---|---|---|---|---|---|---|
| *) phosphorus level in soil was provided | | | | | | | | | |

### Conclusions:

Following effects were observed:
- the stimulating effect of the bioformulation produced based on granulated oat hull and the applied fungi of the genus *Trichoderma* (WMT), on protein and macronutrients (Ca, K and/or Mg and P) level in plants: lettuce, Chinese cabbage, green beans and carrot in soil having lower organic matter content, at higher dose,
- the stimulating effect of the bioformulation produced based on granulated oat hulls and applied fungi of the genus *Trichoderma* and bacteria *Pseudomonas fluorescens* (WMTP), applied at the higher dose on protein and macronutrients (Ca, K and Mg) level in plants: lettuce and cabbage, on the other hand a significantly high phosphorus level was noticed in lettuce, in soil having lower organic matter content,
- the stimulating effect of the bioformulation produced based on granulated oat hull and the applied fungi of the genus *Trichoderma* (WMTEko) applied at the higher dose on protein and macronutrients level (Ca, Mg) in plants of carrot, in soil having lower organic matter content, however, there was no significant increase in potassium level.

## Claims

1. A bioformulation comprising
a carrier granulate comprising
from 95 to 98 % by weight of granulated oat hull, and
from 1 to 3 % by weight of molasses,
based on total mass of the bioformulation,
and at least one microbiological additive selected from the group comprising at least one fungus of genus *Trichoderma* and at least one bacterium selected from the PGPR group,
wherein granulated oat hull has purity in the range from 98 to 99.9 % and is essentially free of microbiological contamination.

2. The bioformulation according to claim 1, comprising from 1 to 2% of at least one microbiological additive.

3. The bioformulation according to claim 1 or 2, wherein the at least one bacterium from the PGPR group is bacterium of genus *Bacillus* and/or *Pseudomonas.*

4. The bioformulation according to any one of the preceding claims 1-3, wherein the at least one microbiological additive is inoculated in pores of granulated oat hull.

5. The bioformulation according to any one of the preceding claims 1-4, wherein it comprises fungi of the *Trichoderma asperellum* species and bacteria of *Bacillus subtilis* and/or *Pseudomonas fluorescens species* as microbiological additives.

6. The bioformulation according to any one of the preceding claims 1-5, which is a plant growth promoter, phytosanitary formulation, plant protection agent, soil conditioner or biostimulant.

7. A method of production of the bioformulation as defined in any one of the preceding claims 1-6, **characterized by** the steps of:
a. preparation of suspension of the at least one microbiological additive selected from the group comprising at least one fungus of genus *Trichoderma* and/or at least one bacterium of the PGPR group
b. incubation of the suspension obtained in the step (a)
c. application of incubated suspension obtained in the step (b) on a carrier granulate.

8. The method according to claim 7, wherein the suspension of the at least one microbiological additive is obtained in step (a) as a result of proliferation of microorganisms on selective culture media.

9. The method according to any one of the preceding claims 7-8, wherein in step (b) incubation of the suspension is carried out in a shaker for about 72 hours for bacteria and for about 12 to about 14 days for fungi of genus *Trichoderma.*

10. The method according to any one of the preceding claims 7-9, wherein in step (c) the incubated suspension is applied on the carrier granulate by spraying or atomization.

11. The method according to any one of the preceding claims 7-10, including an additional step (d) - incubation and drying of the carrier granulate coated with the suspension, carried out after step (c).

12. A method of delivering a bioformulation to soil, **characterized in that** the bioformulation as defined in any one of claims 1-5 is provided using sidedress application in amount of 400 to 800 kg/ha by means of a point seeder when sowing seeds into the soil, by placing the bioformulation at a distance not more than 5 cm below the seed line or to the side of the seed line.

13. A granulated carrier composition comprising
from 97 to 99 % by weight of granulated oat hull and
from 1 to 3 % by weight molasses,
based on total mass of the carrier granulate,
wherein granulated oat hull has purity in range from 98 to 99,9 % and is essentially free of microbiological contamination.

14. A method of production of granulated carrier composition according to claim 12, comprising steps of
a. providing lignocellulosic raw material consisting of a mixture of straw, weeds, seeds of other plants, chaff, ears and hulls of similar components, derived from the threshing of oats,
b. purification of the obtained lignocellulosic material by removing straw, weeds, seeds of other plants, chaff, ears resulting in obtaining a fraction comprising from 98 to 99,9 % oat hulls,
c. optional sterilization of the obtained fraction of oat hulls to remove microbiological contamination,
d. preparation of a mixture by mixing from 97 to 99 % by weight of the fraction of oat hulls and from 1 to 3 % by weight of molasses,
e. granulation of the mixture from step (d) to obtain a carrier granulate.

15. The method according to claim 14, **characterized in that** in the step (d) the hull fraction in form of fine porous particles having particle size not bigger than 4 mm is used.
